# FASCICULE DE BREVET EUROPEEN

(11) **EP 1 191 922 B1**
(45) Date de publication et mention de la délivrance du brevet: **12.04.2006**
(21) Numéro de dépôt: 00953222.7
(22) Date de dépôt: 29.06.2000
(51) Int. Cl.: A61K 8/21, A61K 8/365

(54) **COMPOSITION A BASE DE POLYMERE FLUORE ET DE SEL D'ALPHA-HYDROXY-ACIDE UTILISABLE PAR VOIE TOPIQUE POUR LA PROTECTION DE LA PEAU**
ZUBEREITUNG ZUR TOPISCHEN ANWENDUNG ZUM SCHUTZ DER HAUT AUF BASIS EINES FLUORIERTEN POLYMERS UND EINES SALZES VON ALPHA-HYDROXYSÄUREN
COMPOSITION BASED ON FLUORINATED POLYMER AND ALPHA-HYDROXY-ACID SALT FOR TOPICAL USE FOR SKIN PROTECTION

(30) Priorité: 01.07.1999 FR 9908503
(43) Date de publication de la demande: 03.04.2002
(73) Titulaire: Laboratoire D'Evolution Dermatologique (L.E.D.), 75008 Paris (FR)
(72) Inventeur: ESTANOVE, Cyril, F-92100 Boulogne (FR); ROBERTY-LAUBREAUX, Laurence, F-92100 Boulogne (FR)
(74) Mandataire: L'Helgoualch, Jean
(86) Numéro de dépôt international: PCT/FR2000/001829
(87) Numéro de publication internationale: WO 2001/001923

(56) Documents cités:
- EP-A- 0 803 245
- WO-A-96/40044
- DE-A- 4 327 679
- US-A- 3 317 396
- US-A- 3 932 614
- DATABASE CAPLUS [en ligne] STN INTERNATIONAL; AN1994:663321, 28 novembre 1994 (1994-11-28) TANAKA, Y.: "Cosmetic powders containing silicea and fluorine compound-coated powders" XP002130867

## Description

La présente invention concerne une nouvelle composition cosmétique et/ou dermatologique, et plus particulièrement une composition comprenant en combinaison un polymère fluoré et un sel d'ammonium d'alpha-hydroxy acide, utilisable par application topique pour le traitement et la prévention des affections de la peau.

La peau comprend trois parties, l'épiderme, le derme et l'hypoderme, dont chacune a des propriétés spécifiques faisant de cet ensemble un véritable organe réagissant et s'adaptant aux conditions de son environnement. Le rôle de l'épiderme, couche la plus externe, est donc fondamental pour assurer la protection et le maintien d'une bonne trophicité.

On sait que, pour être acceptées par les utilisateurs, les compositions cosmétiques et dermatologiques destinées au traitement des affections de la peau par application topique doivent être agréables à utiliser et présenter de bonnes propriétés physiques, notamment de consistance et d'onctuosité, tout en garantissant une efficacité satisfaisante.

Pour répondre à ces exigences, on a déjà proposé des compositions contenant des silicones, ou encore des résines perfluorocarbonées ou des polytétrafluoroéthylènes (PTFE) comme dans le brevet US 3.932.614, à titre de lubrifiant. Des polymères fluorés sont aussi incorporés dans d'autres compositions, par exemple une composition dentaire anticalcaire comme dans le brevet US 3.317.396 ou un stick pour les lèvres comme dans la demande WO 96.40044 où une silicone fluorée ayant une viscosité déterminée peut être encapsulée dans une cire ou une huile avec laquelle il est incompatible. Le brevet EP 803.245 décrit un gel aqueux solide à base de polysaccharides contenant un agent hydratant et une phase pulvérulente, dont la structure peut être modifiée de manière classique par addition d'esters d'acides gras et d'hydroxy-acides.

Cette nécessité de mettre à la disposition des utilisateurs des compositions conciliant facilité d'utilisation et efficacité est plus grande encore pour certaines catégories d'utilisateurs ayant une peau très sensible ou confrontés à des risques résultant de leurs activités personnelles ou professionnelles, par exemple les peintres, les ouvriers en bâtiment, les coiffeurs, etc., qui manipulent souvent des produits agressifs ou détergents, ou encore les personnels médicaux qui doivent se laver les mains fréquemment et dont la peau est souvent en contact avec des substances telles que des alcools, des éthers, des produits désinfectants, etc.

Il est cependant difficile de concilier l'efficacité et l'aspect pratique d'application d'une composition destinée à assurer la prévention et/ou la correction des affections dermatologiques considérées.

La présente invention a pour objet une composition cosmétique et/ou dermatologique, administrable par voie topique, présentant d'excellentes qualités d'utilisation et permettant de renforcer la protection de la peau contre les agressions extérieures et de favoriser le processus de réparation de diverses affections cutanées.

Ainsi, la présente invention a pour objet une composition cosmétique et/ou dermatologique administrable par voie topique, qui se distingue en ce qu'elle comprend en combinaison un polymère fluoré et un sel d'ammonium d'alpha-hydroxy-acide, en association, le cas échéant, avec des supports et excipients acceptables sur le plan cosmétique et/ou dermatologique.

De préférence, la composition de l'invention comprend une combinaison de polymère fluoré et de lactate d'ammonium.

Conformément à la présente invention, il est tout particulièrement avantageux d'incorporer de l'aloe vera dans la composition, en combinaison avec le polymère fluoré et le sel d'ammonium d'alpha-hydroxy-acide.

La composition conforme à la présente invention contient de préférence entre 1 et 30 % en poids de polymère fluoré, entre 1 et 20 % en poids de sel d'ammonium d'alpha-hydroxy-acide, et entre 1 et 25 % en poids d'aloe vera. Plus particulièrement, la composition contient entre 3 et 15 % en poids de polymère fluoré, entre 5 et 15 % en poids de sel d'ammonium d'alpha-hydroxy-acide, et entre 2 et 8 % en poids d'aloe vera.

Les polymères fluorés utilisés dans la présente invention sont parfaitement inertes vis-à-vis des ingrédients, excipients et principes actifs couramment utilisés dans les compositions cosmétiques et dermatologiques, et n'entraînent pas de phénomène d'intolérance ou de toxicité vis-à-vis de l'épiderme humain ou animal. Ces polymères fluorés se présentent généralement sous forme d'huile ou de résine, par exemple une poudre blanche constituée de fines particules sensiblement sphériques, de très faible granulométrie, de dimension moyenne inférieure à 50 µm, et de préférence inférieure à 20 µm, avec un faible degré de dispersion autour de la valeur moyenne.

Suivant une forme préférentielle de réalisation, le polymère fluoré utilisé dans l'invention est une résine fluorée ou polytétrafluoroéthylène (PTFE), insoluble dans l'eau et les alcools, possédant une granulométrie inférieure à 10 µm, qui peut être proche de 5 µm environ, ainsi qu'une densité relativement élevée, supérieure à 2.

A titre d'exemple de polymère fluoré utilisable dans la présente invention, on peut citer, outre le polytétrafluoroéthylène, des polymères et copolymères de chlorotrifluoroéthylène, d'hexafluoropropylène, de fluorure de vinylidène, ou d'éther perfluoré de méthyle et d'isopropyle, des copolymères de tétrafluoroéthylène avec l'hexafluoroéthylène ou l'éther de perfluorovinyle, ou des copolymères de perfluoropropylène et d'éthylène, etc. Ces polymères ont généralement un poids moléculaire supérieur à 100.000.

Comme indiqué ci-dessus, la composition suivant l'invention contient un sel d'ammonium d'alpha-hydroxy-acide, tel qu'un lactate d'ammonium, en combinaison avec la résine fluorée telle qu'un polytétrafluoroéthylène (PTFE). Il est également avantageux d'incorporer de l'aloe vera dans la composition.

Le sel d'ammonium d'alpha-hydroxy-acide utilisé dans l'invention, et en particulier le lactate d'ammonium, possède des propriétés hydratantes, eutrophiantes et kératorégulatrices. De plus, le polymère fluoré utilisé en combinaison avec le sel d'ammonium d'alpha-hydroxy-acide vient doubler extérieurement et de façon uniforme la couche la plus externe du stratum corneum, permettant ainsi, par un effet mécanique non occlusif, protecteur et lubrifiant, de préserver, renforcer ou restaurer l'effet réservoir de la couche cornée, d'ammonium d'alpha-hydroxy-acide, qui pourra être relargué de manière progressive, continue et prolongée.

Il en résulte, outre une très bonne hydratation, une optimisation des fonctions physiologiques de l'épiderme, apportant une plus grande résistance et une meilleure plasticité ainsi qu'une meilleure absorption des principes actifs qui peuvent être incorporés dans la composition.

Les compositions peuvent se présenter sous forme de crèmes, émulsions huile-dans-eau ou émulsions eau-dans-huile, laits, gels, lotions, masques, sticks ou pommades, et peuvent être utilisées en particulier pour le traitement d'affections dermatologiques telles que pulpites, perlèches et toutes inflammations cutanées, et pour la protection de la peau contre les diverses agressions résultant généralement de l'environnement des utilisateurs, par exemple les agressions climatiques (vent, froid, pluie, etc.), chimiques (détergents) ou la pollution.

La composition de l'invention se révèle particulièrement utile et efficace pour assurer les soins quotidiens de la peau, en particulier chez les sujets à peau fragile ou exposés à des risques d'agression ou d'affections diverses.

La composition suivant l'invention peut contenir d'autres substances connues pour exercer une activité bénéfique complémentaire, et, comme indiqué ci-dessus, il est tout particulièrement avantageux d'incorporer dans la composition des alpha-hydroxy acides qui facilitent l'exfoliation des cellules à la surface de l'épiderme. On peut aussi incorporer dans la composition, des céramides, des antiperspirants, des anti-infectieux, des antimycosiques, des antiviraux, des vitamines telles que la vitamine C ou la vitamine A, ou encore du tocophérol.

Les compositions conformes à la présente invention sont destinées à être administrées par voie topique et comprennent donc, outre les composants essentiels indiqués ci-dessus, des excipients, additifs et supports usuels acceptables sur le plan dermatologique et cosmétique, sélectionnés en fonction de leurs propriétés connues et de la forme galénique choisie. Ainsi, on peut incorporer dans la composition des conservateurs, des émulsifiants, des épaississants, des gélifiants, des antioxydants, des agents hydratants additionnels, des tensioactifs, des parfums et divers additifs destinés à améliorer les propriétés physiques de la composition. On peut encore avantageusement incorporer des filtres ou écrans solaires choisis en fonction du degré de protection recherché.

Par exemple, les émulsifiants peuvent être choisis parmi ceux couramment employés dans la technique tels qu'un Polysorbate (par exemple le Tween 60® ou le Tween 80®), un ester de sorbitan tel que le stéarate ou le laurate de sorbitan, ou des dérivés d'acide stéarique comme le stéarate de PEG 100®, un stéarate de propylène glycol, un stéarate de polyéthylène glycol, un stéareth ou un cétéareth, ou encore un ester de sucre.

Les conservateurs usuels de la technique des compositions dermatologiques ou cosmétologiques peuvent être utilisés dans l'invention, et par exemple l'acide benzoïque et un p-hydroxybenzoate d'alkyle tel que les p-hydroxy-benzoates de méthyle et de propyle (Méthylparaben et Propylparaben), ou l'imidazolidinyl urée.

L'agent hydratant additionnel peut être par exemple un polyol, le sorbitol, les polyacrylates et polyméthacrylates de glycéryle, le glycérol ou des dérivés de glycérol. On peut aussi ajouter des émollients tels qu'un malate d'alkyle, l'isohexadécane, des triglycérides d'acide caprique ou caprylique, etc.

Les gélifiants peuvent être choisis par exemple parmi les polyacrylamides, les dérivés de cellulose comme l'hydroxypropyl cellulose, ou les gommes naturelles.

La composition suivant la présente invention présente l'avantage de procurer une bonne lubrification de surface, sans utilisation de matières grasses en quantités importantes, une excellente acceptabilité par les utilisateurs qui peuvent exercer leurs activités habituelles sans être aucunement gênés par la composition appliquée sur leur peau, et une très bonne durée d'action, et plus particulièrement une protection prolongée.

Les exemples suivants illustrent plus en détail l'invention. Dans tous les exemples de compositions qui suivent, les parties sont exprimées en poids, sauf indication contraire.

### Exemple 1

On prépare une crème (émulsion huile-dans-eau) ayant la composition suivante exprimée en parties en poids :

| | | |
|---|---|---|
| Polytétrafluoroéthylène (PTFE) | | 15,00 |
| lactate d'ammonium | | 8,00 |
| aloe vera 20 X | | 5,00 |
| Stéareth 2 | | 3,00 |
| Stéareth 21 | | 2,50 |
| Triglycérides (caprique/caprylique) | | 7,00 |
| Methylparaben | | 0,10 |
| Propylparaben | | 0,15 |
| Ether de stéaryle PPG-15 | | 4,00 |
| alcool cétylique | | 1,00 |
| glycérine | | 5,00 |
| huile de vaseline | | 2,00 |
| triéthanolamine | | 0,05 |
| imidazolidinyl urée | | 0,20 |
| carbomer | | 0,10 |
| eau | q.s.p. | 100,00 |

Pour réaliser cette émulsion, on utilise une poudre blanche de PTFE lipophobe de densité égale à 2,2, insoluble dans l'eau, les alcools, le chlorure de méthylène et l'hexane. Le diamètre moyen des particules de PTFE est de 11 µm, et 90% des particules ont un diamètre inférieur à 20 µm. Ce PTFE est incorporé dans une émulsion huile-dans-eau en utilisant comme émulsionnant un mélange de Stéareth 2 et de Stéareth 21, en opérant à chaud, à une température d'environ 75°C. Un épaississant incorporé dans la phase aqueuse empêche toute sédimentation du PTFE et assure une bonne suspension.

### Exemple 2 (non selon l'invention)

On prépare un stick ayant la composition indiqué ci-après :

| | | |
|---|---|---|
| Perfluoropolyméthylisopropyl éther | | 1,00 |
| lactate d'alkyle | | 15,00 |
| aloe vera (gel) | | 5,00 |
| alcool cétylique | | 10,00 |
| beurre de karité | | 5,00 |
| paraffine | | 10,00 |
| ozokerite | | 10,00 |
| cire d'abeille | | 25,00 |
| propylparaben | | 0,10 |
| glycyrrhizate d'ammonium | | 0,10 |
| parfum | | 0,30 |
| huile de vaseline | q.s.p. | 100,00 |

### Exemple 3

On prépare une crème analogue à celle de l'Exemple 1 mais en utilisant un PTFE de granulométrie plus fine, le diamètre moyen des particules étant de 3 µm, 90% des particules ayant un diamètre inférieur à 6 µm.

L'incorporation des particules de PTFE est faite à chaud dans la phase grasse.

| | | |
|---|---|---|
| Polytétrafluoroéthylène (PTFE) | | 10,00 |
| lactate d'ammonium | | 10,00 |
| aloe vera 20 X | | 3,00 |
| alkyle malate | | 4,00 |
| Triglycérides (caprique/caprylique) | | 1,00 |
| Stéareth 2 | | 3,00 |
| Stéareth 21 | | 2,50 |
| isohexadécane | | 2,00 |
| alcool cétylique | | 1,00 |
| propylparaben | | 0,10 |
| méthylparaben | | 0,15 |
| diméthicone | | 4,00 |
| glycérine | | 5,00 |
| imidazolidinyl urée | | 0,20 |
| esters de tri-isostéarine PEG-6 | | 4,00 |
| xanthane | | 0,30 |
| eau | q.s.p. | 100,00 |

### Exemple 4

On prépare une crème (émulsion eau-dans-huile) ayant la composition indiquée ci-après :

| | | |
|---|---|---|
| Polytétrafluoroéthylène (PTFE) | | 10,00 |
| lactate d'ammonium | | 8,00 |
| aloe vera 20 X | | 5,00 |
| Triglycérides (caprique/caprylique) | | 4,00 |
| PEG 30 dipolyhydroxystéarate | | 2,00 |
| malate d'alkyle | | 5,00 |
| lactate d'alkyle | | 4,00 |
| propylparaben | | 0,10 |
| méthylparaben | | 0,15 |
| glycérine | | 5,00 |
| sulfate de magnésium | | 0,80 |
| imidazolidinyl urée | | 0,20 |
| parfum | | 0,20 |
| eau | q.s.p. | 100,00 |

On constate, par examen au microscope, que les particules de PTFE sont uniformément réparties dans la suspension sans former le moindre agrégat. Les émulsions préparées comme indiqué ci-dessus présentent une excellente souplesse et de bonnes propriétés de douceur lors de l'application sur la peau.

Les compositions conformes à la présente invention, décrites ci-dessus, présentent d'excellentes propriétés protectrices de la peau, tout en préservant la perméabilité sélective de la couche cornée et son effet réservoir, assurant une efficacité maximale des principes actifs utilisés.

## Revendications

1. Composition cosmétique et/ou dermatologique administrable par voie topique, **caractérisée en ce qu'**elle comprend un polymère fluoré en combinaison avec un sel d'ammonium d'alpha-hydroxy-acide.

2. Composition selon la revendication 1, **caractérisée en ce qu'**elle contient en outre de l'aloe vera.

3. Composition selon la revendication 1, **caractérisée en ce que** le sel d'ammonium est le lactate d'ammonium.

4. Composition selon l'une quelconque des revendications précédentes, **caractérisée en ce que** la résine fluorée est choisie parmi le polytétrafluorcéthylène, des polymères et copolymères de chlorotrifluoroéthylène, d'hexafluoropropylène, de fluorure de vinylidène, ou d'éther perfluoré de méthyle et d'isopropyle, des copolymères de tétrafluoroéthylène avec l'hexafluoroéthylène ou l'éther de perfluorovinyle, ou des copolymères de perflucropropylène et d'éthylène.

5. composition selon la revendication 4, **caractérisée en ce que** la résine fluorée est un polytétrafluoroéthylène de granulométrie moyenne inférieure à 50 *µ*m.

6. Composition selon la revendication 5, **caractérisée en ce que** le diamètre moyen des particules de polytétrafluoroéthylène est inférieur à 20 *µ*m.

7. Composition selon la revendication 2, **caractérisée en ce qu'**elle contient entre 1 et 30 % en poids de polymère fluoré, entre 1 et 20 % en poids de sel d'ammonium d'alpha-hydroxy-acide, et entre 1 et 25 % en poids d'aloe vera.

8. Composition selon la revendication 7, **caractérisée en ce qu'**elle contient entre 3 et 15 % en poids de polymère fluoré, entre 5 et 15 % en poids de sel d'ammonium d'alpha-hydroxy-acide, et entre 2 et 8 % en poids d'aloe vera.

9. Utilisation d'une combinaison d'un polymère fluoré et d'un sel d'ammonium d'alpha-hydroxy-acide pour la fabrication d'une composition cosmétique et/ou dermatologique administrable par voie topique.

## Patentansprüche

1. Kosmetische und/oder dermatologische Zusammensetzung, die topisch verabreichbar ist, **dadurch gekennzeichnet, dass** sie ein fluoriertes Polymer in Kombination mit einem Ammoniumsalz einer α-Hydroxysäure umfasst.

2. Zusammensetzung nach Anspruch 1, **dadurch gekennzeichnet, dass** sie weiters Aloe Vera umfasst.

3. Zusammensetzung nach Anspruch 1, **dadurch gekennzeichnet, dass** das Ammoniumsalz Ammoniumlactat ist.

4. Zusammensetzung nach einem der vorangehenden Ansprüche, **dadurch gekennzeichnet, dass** das fluorierte Harz aus Polytetrafluorethylen, Polymeren und Copolymeren von Chlortrifluorethylen, Hexafluorpropylen, Vinylidenfluorid oder perfluoriertem Methyl- und Isopropylether, Copolymeren von Tetrafluorethylen mit Hexafluorethylen oder Perfluorvinylether oder Copolymeren von Perfluorpropylen mit Ethylen ausgewählt ist.

5. Zusammensetzung nach Anspruch 4, **dadurch gekennzeichnet, dass** das fluorierte Harz Polytetrafluorethylen mit einem mittleren Teilchendurchmesser von 50 µm ist.

6. Zusammensetzung nach Anspruch 5, **dadurch gekennzeichnet, dass** der mittlere Durchmesser der Polytetrafluorethylen-Teilchen unter 20 µm liegt.

7. Zusammensetzung nach Anspruch 2, **dadurch gekennzeichnet, dass** sie zwischen 1 und 30 Gew.-% fluoriertes Polymer, zwischen 1 und 20 Gew.-% des Ammoniumsalzes einer α-Hydroxysäure und zwischen 1 und 25 Gew.% Aloe Vera enthält.

8. Zusammensetzung nach Anspruch 7, **dadurch gekennzeichnet, dass** sie zwischen 3 und 15 Gew.-% fluoriertes Polymer, zwischen 5 und 15 Gew.-% des Ammoniumsalzes einer α-Hydroxysäure und zwischen 2 und 8 Gew.-% Aloe Vera enthält.

9. Verwendung einer Kombination aus einem fluorierten Polymer und dem Ammoniumsalz einer α-Hydroxysäure zur Herstellung einer kosmetischen und/oder dermatologischen Zusammensetzung, die topisch verabreichbar ist.

## Claims

1. Cosmetic and/or dermatologic composition for topical administration, **characterized in that** it comprises a fluorinated polymer combined with an alpha-hydroxy-acid ammonium salt.

2. Composition according to claim 1, **characterized in that** it further contains aloe vera.

3. Composition according to claim 1, **characterized in that** the ammonium salt is ammonium lactate.

4. Composition according to any of the preceding claims, **characterized in that** the fluorinated resin is selected from polytetrafluoroethylene, polymers and copolymers of chlorotrifluoroethylene, hexafluoropropylene, vinylidene fluoride, or methyl isopropyl perfluorinated ether, copolymers of tetrafluoroethylene and hexafluoroethylene or perfluorovinyl ether, or copolymers of perfluoropropylene and ethylene.

5. Composition according to claim 4, **characterized in that** the fluorinated resin is a polytetrafluoroethylene with a mean particle size lower than 50 µm.

6. Composition according to claim 5, **characterized in that** the mean particle size of polytetrafluoroethylene is lower than 20 µm.

7. Composition according to claim 2, **characterized in that** it contains from 1 to 30% by weight of fluorinated polymer, from 1 to 20% by weight of alpha-hydroxy-acid ammonium salt, and from 1 to 25% by weight of aloe vera.

8. Composition according to claim 7, **characterized in that** it contains from 3 to 15% by weight of fluorinated polymer, from 5 to 15% by weight of alpha-hydroxy-acid ammonium salt, and from 2 to 8% by weight of aloe vera.

9. The use of a combination of a fluorinated polymer and an alpha-hydroxy-acid ammonium salt in the manufacture of a cosmetic and/or dermatologic composition for topical administration.
